(19) Europäisches Patentamt
European Patent Office
Office européen des brevets

(11) **EP 1 857 095 A1**

(12) **DEMANDE DE BREVET EUROPEEN**

(43) Date de publication:
**21.11.2007 Bulletin 2007/47**

(21) Numéro de dépôt: **07106769.8**

(22) Date de dépôt: **24.04.2007**

(51) Int Cl.:
*A61K 8/06* (2006.01)   *A61K 8/894* (2006.01)
*A61K 8/90* (2006.01)   *A61K 8/44* (2006.01)
*A61K 8/34* (2006.01)   *A61Q 1/00* (2006.01)
*A61Q 5/02* (2006.01)   *A61Q 19/10* (2006.01)
*A61Q 19/00* (2006.01)

(84) Etats contractants désignés:
**AT BE BG CH CY CZ DE DK EE ES FI FR GB GR
HU IE IS IT LI LT LU LV MC MT NL PL PT RO SE
SI SK TR**
Etats d'extension désignés:
**AL BA HR MK YU**

(30) Priorité: **17.05.2006  FR 0604406**

(71) Demandeur: **L'ORÉAL**
**75008 Paris (FR)**

(72) Inventeur: **Inoue, Mika**
**TOKYO 206-0002 (JP)**

(74) Mandataire: **Rasson, Catherine**
**L'OREAL - D.I.P.I.**
**25-29 Quai Aulagnier**
**92600 Asnières (FR)**

(54) **Emulsion fine huile dans eau**

(57)   La présente invention se rapporte à une composition pour application topique sous forme d'émulsion huile-dans-eau, caractérisée en ce qu'elle contient (1) un système émulsionnant contenant au moins un tensioactif siliconé de poids moléculaire égal ou supérieur à 10000, au moins un co-tensioactif non ionique et au moins un co-tensioactif anionique, et (2) au moins un alcool monohydrique comportant de 2 à 6 atomes de carbone, et en ce que les huiles de la phase huileuse sont solubles dans l'alcool monohydrique.

Ces compositions sont des émulsions fines et elles présentent une bonne stabilité et de bonnes propriétés cosmétiques. Elles peuvent être utilisées dans de nombreuses applications cosmétiques et dermatologiques.

EP 1 857 095 A1

**Description**

La présente invention se rapporte à une composition pour application topique sous forme d'émulsion huile-dans-eau fine, son procédé de préparation et son utilisation notamment pour le traitement, le soin, le maquillage et/ou le nettoyage de la peau, des phanères (cheveux, cils, ongles) et/ou des muqueuses. La composition peut être notamment une composition cosmétique et/ou dermatologique.

Pour diverses raisons liées en particulier à un meilleur confort d'utilisation (douceur, émollience et autres), les compositions cosmétiques ou dermatologiques actuelles se présentent le plus souvent sous la forme d'une émulsion du type huile-dans-eau (H/E), c'est à dire une émulsion constituée d'une phase continue dispersante aqueuse et d'une phase discontinue dispersée huileuse.

Les émulsions huile dans eau sont stabilisées généralement par des tensioactifs émulsionnants appropriés aux émulsion H/E, qui grâce a leur structure amphiphile, se placent à l'interface phase huileuse / phase aqueuse, et stabilisent ainsi les gouttelettes d'huile dispersées dans la phase aqueuse. Malgré la présence d'émulsionnants, les émulsions peuvent avoir tendance à se déphaser. Pour résoudre les problèmes de stabilité des émulsions H/E classiques, plusieurs solutions sont connues. Par exemple, on peut augmenter la viscosité de la phase continue (phase aqueuse), par un ou des gélifiants hydrosolubles. Toutefois, cette solution ne convient pas pour obtenir des produits fluides ou très liquides comme les produits constituant des toniques et des lotions, appelés aussi « cosmetic water ».

Une autre solution envisageable pour stabiliser les émulsions huile dans eau fluide sans ajout d'épaississant ou avec un très faible taux d'épaississant, consiste à faire des émulsions H/E dites « ultrafines », dans lesquelles la taille moyenne des globules constituant la phase huileuse est comprise dans des limites bien déterminées, à savoir entre 50 et 1000 nm.

Plusieurs techniques d'émulsions ultrafines sont connues, comme par exemple:

- les nanoémulsions, comme décrites par exemple dans les documents EP-A-728460, EP-A-780114, EP-A-1010413, EP-A-1010414, EP-A-1010415, EP-A-1010416, EP-A-1013338, EP-A-1016453, EP-A-1018363, EP-A-1020219, EP-A-1025898, EP-A-1172077. L'inconvénient de cette technologie est la nécessité d'utiliser une énergie mécanique et notamment un homogénéisateur à haute pression, ce qui rend le procédé compliqué ;
- les émulsions PIT, comme décrit par exemple dans le document EP-A-1297824, qui ne nécessitent pas d'énergie mécanique la une température du domaine d'inversion de phase. Ce procédé présente l'inconvénient de limiter le type de composition ; Ainsi, par exemple, on peut difficilement modifier les taux de polyol et d'alcool car cela entraîne une modification de la température d'inversion de phase. Un autre inconvénient de cette technologie est de ne pas pouvoir diminuer la phase huileuse à moins de 5%. Or, dans le cas des lotions et toniques, on peut souhaiter avoir des émulsions H/E contenant beaucoup d'eau et moins de 5 % en poids d'huiles.

A côté de ces techniques de préparation d'émulsions ultra-fines, on connaît les microémulsions qui peuvent aussi être fluides. Les microémulsions, comme décrit par exemple dans le document EP-A-0774482, ne sont pas à proprement parler des émulsions mais ce sont des dispersions thermodynamiquement stables constituées de micelles de lipides amphiphiles gonflées par de l'huile, cette huile étant en général à chaîne très courte (par exemple hexane ou décane) et étant par ailleurs solubilisée grâce à la présence conjointe d'une quantité importante de tensioactifs et de co-tensioactifs formant les micelles. La taille des micelles gonflées est très petite en raison de la faible quantité d'huile qu'elles peuvent solubiliser. Cette très petite taille des micelles est la cause de leur transparence. Les microémulsions se forment spontanément par simple mise en contact des constituants (eau, huiles et tensioactifs) sans apport d'énergie mécanique autre qu'une simple agitation magnétique et quel que soit l'ordre d'ajout des constituants. Les inconvénients majeurs des microémulsions sont liés à leur forte proportion en tensioactifs, conduisant à des intolérances et entraînant un toucher collant lors de l'application sur la peau. Par ailleurs, leur domaine de formulation est en général très étroit. Ces microémulsions ne sont donc pas des émulsions fines et ne peuvent pallier les inconvénients des émulsions fines décrites ci-dessus.

Il subsiste donc le besoin de réaliser des émulsions H/E fines et fluides et qui soient stables et qui soient obtenues avec des procédés moins coûteux et moins complexes que ceux de l'art antérieur, c'est à dire des procédés ne nécessitant pas d'apport d'énergie, que cette énergie soit mécanique ou thermique, et donc sans passer par des températures élevées et sans nécessiter de matériel apportant beaucoup d'énergie, ces procédés étant tels qu'ils n'ont pas d'incidence sur la stabilité chimique des composés constituant la composition.

La demanderesse a trouvé de façon surprenante la possibilité de réaliser des émulsions fines sans apport d'énergie et sans chauffage, grâce à un choix particulier de tensioactifs et de co-tensioactifs, et à la présence d'alcool.

L'invention se rapporte aussi aux utilisations de la dite composition, notamment dans le domaine cosmétique, telle quelle ou sous forme d'article imprégné de ladite composition.

La présente invention a donc pour objet une composition sous forme d'émulsion huile-dans-eau, comprenant une phase huileuse dispersée dans une phase aqueuse, caractérisée en ce qu'elle contient (1) un système émulsionnant contenant au moins un tensioactif siliconé de poids moléculaire égal ou supérieur à 10000, au moins un co-tensioactif non ionique et au moins un co-tensioactif anionique, et (2) au moins un alcool monohydrique comportant de 2 à 6 atomes de carbone, et en ce que la ou les huiles de la phase huileuse sont solubles dans l'alcool monohydrique.

La composition revendiquée présente les avantages d'avoir une bonne stabilité, même en l'absence d'épaississants dans la phase aqueuse et même à très faible taux de huile (par exemple 1 à 2 %), d'être facile à fabriquer (agitation classique en vitesse lente, pas de nécessité de chauffer, ni d'homogénéiser sous haute pression), de permettre la variation des taux d'alcools et des polyols, d'avoir une très bonne qualité cosmétique, et de présenter une bonne tolérance oculaire et cutanée.

La composition est notamment destinée à une application topique. On entend ici par « application topique » une application externe sur les matières kératiniques, que sont notamment la peau, le cuir chevelu, les cils, les sourcils, les ongles, les cheveux et/ou les muqueuses. La composition étant destinée à une application topique, elle comprend un milieu physiologiquement acceptable. On entend par « milieu physiologiquement acceptable », un milieu compatible avec la peau, les lèvres, le cuir chevelu, les cils, les yeux, les ongles et/ou les cheveux. La composition peut constituer notamment une composition cosmétique ou dermatologique.

La composition selon l'invention est stable à tout pH. Toutefois, pour une application topique, il est préférable que la composition ait un pH allant de 3 à 9 et mieux de 3,5 à 8,5.

La composition revendiquée présente des globules de la phase huileuse, ayant une taille moyenne en nombre (diamètre) allant de 50 à 1000 nm, de préférence de 100 nm à 500 nm et plus particulièrement de 100 à 300 nm, cette taille moyenne étant une taille moyenne en intensité, mesurée par diffusion quasi-élastique de la lumière, par exemple par l'appareil Model Instrument 90+ de la société Brookhaven Instruments Corporation.

Selon la taille des globules de phase huileuse, l'aspect visuel de la composition selon l'invention peut aller de l'aspect translucide ou opalescent à un aspect blanc. Les émulsions fines de l'invention ont généralement une turbidité allant de 100 à 900 NTU et de préférence de 200 à 600 NTU, turbidité mesurée au turbidimètre portatif HACH - Modèle 2100 P.

Système émulsionnant

La composition selon l'invention contient un système émulsionnant contenant au moins un tensioactif siliconé de poids moléculaire égal ou supérieur à 10000, au moins un co-tensioactif non ionique, et au moins un co-tensioactif anionique.

Le système émulsionnant est présent en une quantité pouvant aller par exemple de 0,07 à 5 % en poids, de préférence de 0,1 à 5 % en poids et mieux de 0,2 à 3 % en poids par rapport au poids total de la composition.

A. Tensioactif siliconé

On entend par « tensioactif siliconé », un composé siliconé comportant au moins une chaîne oxyalkylénée, notamment comportant au moins une chaîne oxyéthylénée (-OCH$_2$CH$_2$-) et/ou oxypropylénée (-OCH$_2$CH$_2$CH$_2$-).

Le tensioactif siliconé utilisé dans la présente invention doit être soluble ou dispersible dans l'alcool monohydrique et noyamment dans l'éthanol. Il est en outre de préférence choisi parmi les tensioactifs liquides à température ambiante.

Les tensioactifs siliconés appropriés sont ceux ayant un poids moléculaire égal ou supérieur à 10000, et ils peuvent être choisis notamment parmi les polydiméthylsiloxanes comportant à la fois des groupements oxyéthylénés et des groupements oxypropylénés. On peut citer par exemple le polydiméthylsiloxane à terminaison oxyéthylène/ oxypropylène commercialisé en mélange avec des triglycérides d'acides caprylique/caprique sous la dénomination Abil care 85 par la société Goldschmidt (nom INCI: BIS-PEG/PPG-16/16 PEG/PPG-16/16 Dimethicone / Caprylic/Capric Triglyceride), le polydiméthylsiloxane à groupement alpha-omega polyéther (OE / OP: 40 / 60), commercialisé sous la dénomination Abil B8832 par la société Goldschmidt (nom INCI : BIS-PEG/PPG-20/20 Dimethicone), le polydiméthylsiloxane oxyéthyléné oxypropyléné commercialisé sous la dénomination Abil B88184 par la société Goldschmidt (nom INCI: PEG/PPG-20/6 Dimethicone) et le polydiméthyl / méthylsiloxane oxyéthyléné oxypropyléné commercialisé sous la dénomination Abil B8852 par la société Goldschmidt (nom INCI: PEG/PPG-4/12 Dimethicone), et leurs mélanges.

Selon un mode préféré de réalisation de l'invention, le tensioactif siliconé est l'Abil care 85.

La quantité de tensioactif(s) siliconé(s) va de préférence de 0,05 à 4 % en poids par rapport au poids total de la composition.

Selon un mode préféré de réalisation de l'invention, le rapport en poids de la quantité d'huile(s) sur la quantité de tensioactif(s) siliconé(s) va de 0,5 à 20 et mieux de 1 à 10, et le rapport en poids des quantités de tensioactif(s) siliconé(s) et d'huile(s) sur la quantité d'alcool(s) monohydrique(s) va de 0,01 à 1, mieux de 0,05 à 0,3 et encore mieux de 0,1 à 0,2.

Dans la présente demande, on entend par « quantité d'huiles » la quantité totale des huiles présentes dans la composition.

Par ailleurs, selon un mode préféré de réalisation de l'invention, le rapport en poids de la quantité de co-tensioactif(s) anionique(s) sur la quantité de tensioactif(s) siliconé(s) va de 0,01 à 1 et mieux de 0,05 à 0,5.

B. Co-tensioactif non ionique

La composition selon l'invention contient au moins un co-tensioactif non ionique qui est choisi de préférence parmi les tensioactifs non ioniques ayant un HLB inférieur ou égal à 15.

On calcule la balance HLB (balance hydrophile-lipophile) d'un tensioactif émulsionnant suivant la formule suivante :

$$HLB = 20 \times \frac{m \text{ hydrophile}}{m \text{ totale du TA}}$$

dans laquelle m hydrophile représente le poids du groupement hydrophile (soit la partie polaire) et m totale du TA représente le poids total du tensioactif.

Comme tensioactifs non ioniques pouvant être utilisés comme co-tensioactifs dans la présente demande, on peut citer notamment les polycondensats d'oxyde d'éthylène et d'oxyde de propylène, et plus particulièrement les copolymères consistant en des blocs polyéthylène glycol et polypropylène glycol, comme par exemple les polycondensats tribloc polyéthylène glycol / polypropylène glycol / polyéthylène glycol. Ces polycondensats tribloc ont par exemple la formule chimique suivante :

$$H\text{-}(O\text{-}CH_2\text{-}CH_2)_a\text{-}(O\text{-}CH(CH_3)\text{-}CH_2)_b\text{-}(O\text{-}CH_2\text{-}CH_2)_a\text{-}OH,$$

où a va de 2 à 150, et b va de 1 à 100 ; de préférence a va de 10 à 130 et b va de 20 à 80.

Le co-tensioactif non ionique a de préférence un poids moléculaire moyen en poids allant de 1000 à 15000, et de mieux allant de 1500 à 15000, et en particulier allant de 1500 à 10000, et encore mieux allant de 1500 à 5000.

Comme polycondensats, on peut citer notamment les polycondensats tribloc polyéthylène glycol / polypropylène glycol / polyéthylène glycol, vendus sous les dénominations "SYNPERONIC" par la société Uniqema, comme le condensat d'oxyde d'éthylène, d'oxyde de propylène et d'oxyde d'éthylène (13 OE /30 OP /13 OE) (PM: 2900) commercialisé sous la dénomination SYNPERONIC PE/L 64 (Poloxamer 184), le condensat d'oxyde d'éthylène, d'oxyde de propylène et d'oxyde d'éthylène (8 OE /30 OP /8 OE) (PM: 2500) commercialisé sous la dénomination SYNPERONIC PE/L 62 (nom INCI : Poloxamer 182), le condensat d'oxyde d'éthylène, d'oxyde de propylène et d'oxyde d'éthylène (6 OE /67 OP /6 OE) (PM: 4400) commercialisé sous la dénomination SYNPERONIC PE/L 121 (nom INCI: Poloxamer 401), le condensat d'oxyde d'éthylène, d'oxyde de propylène et d'oxyde d'éthylène (46 OE /16 OP /46 OE) (PM: 5000) commercialisé sous la dénomination SYNPERONIC® PE/F38 (nom INCI : Poloxamer 108), le condensat d'oxyde d'éthylène, d'oxyde de propylène et d'oxyde d'éthylène (128 OE /54 OP /128 OE) (PM: 14000) commercialisé sous la dénomination SYNPERONIC® PE/F108 (nom INCI: Poloxamer 338), le condensat d'oxyde d'éthylène, d'oxyde de propylène et d'oxyde d'éthylène (11 OE /21 OP /11 OE) (PM: 2200) commercialisé sous la dénomination SYNPERONIC® PE/ L44 (nom INCI: POLOXAMER 124), le condensat d'oxyde d'éthylène, d'oxyde de propylène et d'oxyde d'éthylène (5 OE /21 OP/5 OE) (PM: 1630) commercialisé sous la dénomination SYNPERONIC® PE/L42 (nom INCI : Poloxamer 122), le condensat d'oxyde d'éthylène, d'oxyde de propylène et d'oxyde d'éthylène (98 OE/67 OP/98 OE) (PM: 12000) commercialisé sous la dénomination SYNPERONIC® PE/F127 (nom INCI: Poloxamer 407), le condensat d'oxyde d'éthylène, d'oxyde de propylène et d'oxyde d'éthylène (97 OE/39 OP/97 OE) (PM: 10800) commercialisé sous la dénomination SYNPERONIC® PE/F88 (nom INCI : Poloxamer 238), et leurs mélanges.

On peut aussi utiliser le condensat d'oxyde d'éthylène, d'oxyde de propylène et d'oxyde d'éthylène (75 OE /30 OP /75 OE) (PM: 8350) commercialisé sous la dénomination LUTROL® F68 » (nom INCI : Poloxamer 188) par la société BASF, et le condensat d'oxyde d'éthylène, d'oxyde de propylène et d'oxyde d'éthylène (13 OE/30 OP/13 OE) (PM: 2900) commercialisé sous la dénomination Pluracare L 64 par la société BASF (nom INCI : Poloxamer 184).

Le co-tensioactif non ionique est de préférence choisi parmi les polycondensats tribloc polyéthylène glycol / polypropylène glycol / polyéthylène glycol (Poloxamer) de HLB égal ou inférieur à 15, notamment le Poloxamer 184, le Poloxamer 182, le Poloxamer 401, et leurs mélanges.

La quantité de co-tensioactif(s) non ionique(s) va de préférence de 0,01 à 0,5 % en poids par rapport au poids total de la composition.

Selon un mode préféré de réalisation de l'invention, le rapport en poids de la quantité de co-tensioactif(s) non ionique(s) sur la quantité d'huiles va de 0,001 à 1 et mieux de 0,005 à 0,5, et encore mieux de 0,01 à 0,2.

## C. Co-Tensioactifs anioniques

Les co-tensioactifs anioniques pouvant être mis en oeuvre dans le cadre de la présente invention sont de préférence choisis dans le groupe comprenant les lipides anioniques neutralisés. On peut les choisir en particulier parmi les lipoaminoacides et leurs sels tels que les acylglutamates mono- et di- sodiques comme le sel disodique de l'acide N-stéaroyl L-glutamique commercialisé sous la dénomination Acylglutamate HS21 P ou Amisoft HS21 P par la société Ajinomoto, le sel monosodique de l'acide N-lauroyl L-glutamique commercialisé sous la dénomination Acylglutamate LS11 ou Amisoft LS11 par la société Ajinomoto, et leurs mélanges.

La quantité de co-tensioactif(s) anionique(s) va de préférence de 0,01 à 0,5 % en poids par rapport au poids total de la composition.

## Alcool monohydrique

La composition selon l'invention contient au moins un alcool monohydrique comportant de 2 à 6 atomes de carbone. Un alcool monohydrique est un oalcool primaire ne comportant qu'une fonction OH.

La quantité d'alcool(s) monohydrique(s) peut aller par exemple de 0.1 à 60 % et de préférence de 5 à 30 % du poids total de la composition.

De préférence, le rapport en poids de la quantité d'alcool(s) monohydrique(s) sur la quantité d'eau va de 0,01 à 0,2, et le rapport en poids de la quantité d'alcool(s) monohydrique(s) sur la quantité d'huile(s) va de 0,05 à 0,2.

Comme alcools monohydriques, on peut citer par exemple l'éthanol, le propanol, le butanol, l'isopropanol, l'isobutanol, et leurs mélanges. L'alcool monohydrique est de préférence l'éthanol.

## Polyols

En plus des alcools monohydriques, la composition peut contenir un ou plusieurs polyols, comme par la glycérine, la polyglycérine, le sorbitol, et les glycols tels que l'isoprène glycol, le 1,3-butylène glycol, le propylène glycol, le dipropylène glycol, et l'hexylène glycol, et leurs mélanges.

La quantité de polyol(s) peut aller par exemple de 1 à 50 %, de préférence de 2 à 30 % et mieux de 5 à 30 % du poids total de la composition.

## Phase huileuse

La phase huileuse est constituée des huiles et de tous les constituants lipophiles pouvant être présents dans la composition de l'invention.

La phase huileuse contient au moins une huile. On entend par "huile" un corps gras liquide à la température ambiante (25°C).

Les huiles utilisées dans la composition de l'invention sont choisies parmi les huiles solubles ou dispersibles dans l'alcool monohydrique. On entend par « solubles » le fait qu'une quantité de 5 % soit solubilisée dans l'alcool monohydrique, c'est-à-dire qu'une solution à 5 % en poids dans l'alcool monohydrique et notamment dans l'éthanol. soit limpide.

La phase huileuse ne contient que des huiles solubles dans l'alcool monohydrique. Toutefois, elle peut aussi contenir des actifs huileux insolubles dans l'alcool monohydrique dans la mesure où ces actifs sont solubles dans l'huile et où, donc, l'ensemble huiles + actifs peut se solubiliser dans l'alcool monohydrique, et notamment l' éthanol.

De façon plus précise, la ou les huiles pouvant être utilisées dans la composition de l'invention sont choisies soit parmi les huiles ayant un poids moléculaire inférieur ou égal à 350, soit parmi les huiles de poids moléculaire supérieur à 350 et ayant une valeur d'IOB (Balance / Inorganique / organique) supérieure ou égale à 0,1.

Le paramètre IOB est connu de l'homme du métier à partir d'un certain nombre de publications comme :

(1) « Prediction of organic compounds by a conceptionnal diagram », A. FUJITA Pharm. Bull 2, 163-173 (1954)
(2) "Organic Analysis" Fujita(1930), publiée par Kaniya Shoten,
(3) « Prediction of Organic Compounds and Organic Conceptional Diagram » A. Fujita (Kagaku-no-Ryoiki 11-10)" (1957), pp.719-725,
(4) "Systematic Organic Qualitative Analysis (Book of Purified Substances)" Fujita et Akatsuka (1970), p.487, publiée par Kazama Shoten,
(5) "Organic Conceptional Diagram, Its Fundamentals and applications", Koda (1984), p.227, publiée par Sankyo Shuppan,

(6) "Design of Emulsion Formulations by use of Organic Conceptional Diagram" (1985), p.98, Yaguchi, publiée par Nippon Emulsion K.K.,

(7) R. H. Ewell, J. M. Harrison, L. Berg.: Ind. Eng. Chem. 36, 871 (1944), (8) EP-A-985404

L'IOB d'un composé correspond au rapport de la valeur inorganique du composé sur la valeur organique du composé :

$$IOB = valeur\ inorganique\ /\ valeur\ organique$$

Pour calculer la valeur organique d'un composé, le groupe méthylène est considéré comme unité et est évalué par le nombre d'atome de carbone. Un atome de carbone ou un groupement -CH3, -CH2-, =CH-, est compté pour une valeur de 20 (valeur sans unité). Les atomes d'hydrogène ne sont pas pris en compte. La présence d'un cycle, d'une ramification, d'une insaturation éthylénique ou acétylénique dans ledit composé organique est prise en compte dans le calcul de la valeur organique du composé selon la valeur organique correspondante connue dans la littérature, notamment en page 167 de la publication (1) citée précédemment.

Pour calculer la valeur inorganique d'un composé, le groupe hydroxyle est pris comme groupe standard pour lequel est attribuée une valeur inorganique de 100. Cette valeur de 100 arbitraire est corrélée à la distance entre la courbe du point d'ébullition de la série des alcanes en fonction du nombre d'atomes de carbone dudit alcane et la courbe du point d'ébullition des monoalcools primaires, saturés, linéaires analogues aux alcanes.

La valeur inorganique (notée Ix) d'un substituant X (c'est à dire de tout atome différent du carbone ou de l'hydrogène, et de tout groupe d'atomes différent des groupes d'atomes formés exclusivement de carbone et/ou d'hydrogène) est déterminée graphiquement. Cette valeur Ix est calculée en déterminant d'une part la température d'ébullition (Eb) d'un alcane linéaire et la température d'ébullition (Ebx) de l'homologue dudit alcane linéaire substitué par le substituant X, puis en calculant la différence $\Delta Tx = Ebx - Eb$, et d'autre part en déterminant la température d'ébullition ($Eb_{OH}$) de l'homologue substitué par un groupe alcool primaire, puis en calculant la différence $\Delta T_{OH} = Eb_{OH} - Eb$. La valeur Ix est égale au rapport de la différence $\Delta Tx$ sur $\Delta T_{OH}$, ledit rapport étant tout multiplié par la valeur inorganique du groupe hydroxyle égale à 100.

$$Ix = \frac{\Delta T_X}{\Delta T_{OH}} \times 100$$

Les valeurs inorganiques de nombreux substituants sont décrites dans la littérature, notamment dans les références citées précédemment. La valeur inorganique d'un composé est calculée en additionnant la valeur inorganique du (ou de tous les) substituant(s) présent(s) dans ledit composé.

Certains substituants ont à la fois une valeur organique et une valeur inorganique, comme l'indique la référence (1) citée précédemment, page 167, comme par exemple les substituants -Cl ou -F.

L'IOB d'un mélange de composés organiques est égal au rapport de la somme des valeurs inorganiques desdits composé organiques du mélange sur la somme des valeurs organiques desdits composés organiques du mélange.

Comme huiles préférées utilisables dans la composition de l'invention, on peut citer par exemple :

- les esters d'acides gras, comme les huiles de formule R[1]COOR[2] dans laquelle R[1] représente le reste d'un acide gras comportant de 8 à 29 atomes de carbone, et R[2] représente une chaîne hydrocarbonée, ramifiée ou non, contenant de 3 à 30 atomes de carbone, comme par exemple l'isononanoate d'isononyle (P.M. = 284), le palmitate d'isopropyle (P.M. = 298), le palmitate de 2-éthyl hexyle (P.M. = 368 ; IOB = 0,128), l'isostéarate d'isopropyle (P.M. = 326 ; IOB = 0,15), les triglycérides d'acides caprylique/caprique ((P.M. = 494 ; IOB = 0,314), les esters du penta-érythritol comme le tétra-octanoate de pentaérythrityle (nom INCI: Pentaerythrityl Tetraethylhexanoate) (P.M. = 640 ; IOB = 0,353), l'adipate de di(2-éthyl hexyle) (P.M. = 370 ; IOB = 0,286), le C12-15 alkyl benzoate (P.M. = 309 ; IOB = 0,184) (Finsolv TN) ;
- les alcools gras ayant de 8 à 26 atomes de carbone, en particulier l'octyldodécanol (P.M. = 300) ;
- l'isohexadecane (P.M. =226), l'isododecane (P.M. = 170) ;
- les huiles de silicone, notamment les huiles de silicone volatiles telles que les cyclopolydiméthylsiloxanes (cyclométhicones) comme la cyclohexasiloxane et la cyclopentasiloxane ((P.M. = 370 ; IOB = 0,4), les silicones phénylées comme les phényltriméthicones (P.M. = 372 ; IOB = 0,212),

- leurs mélanges.

Selon un mode préféré de réalisation de l'invention, la phase huileuse contient une ou plusieurs huiles choisies parmi les phényltriméthicones telles que les produits commercialisés sous les dénominations « Dow Corning 556 Cosmetic Grade Fluid » par la société Dow Corning, « ABIL AV 1000 » et « ABIL AV 20 HS » par la société Goldschmidt, « KF 56 » par la société Shin Etsu, ;les esters du pentaérythritol tels que le tétra-octanoate de pentaérythrityle comme les produits commercialisés sous les dénominations « Nikkol Pentarate 408 » par la société Nikko Chemicals, « Trivent PE-48 » par la société Akzo, « PRIOLUBE 3929 » par la société Uniqema, « Hatcol 5140 » par la société HATCO ; l'octyldodécanol ; l'isohexadecane ; l'isododecane ; les cyclopolydiméthylsiloxanes ; et leurs mélanges.

Selon un mode de réalisation particulièrement préféré, la phase huileuse contient au moins un mélange d'ester de penta-érythritol et de phényltriméthicone, plus particulièrement un mélange de tétra-octanoate de pentaérythrityle et de phenyltrimethicone.

La phase huileuse peut représenter par exemple de 0,5 à 10 % en poids, de préférence de 0,5 à 8 % en poids et mieux de 1 à 5 % en poids par rapport au poids total de la composition.

Phase aqueuse

De manière classique, la phase aqueuse dispersante comprend l'eau, et tous les adjuvants hydrosolubles ou hydrodispersibles.

La composition selon l'invention comprend une quantité importante d'eau. Cette quantité peut aller par exemple de 40 à 90 % en poids, de préférence de 50 à 85 % en poids et mieux de 60 à 85 % en poids par rapport au poids total de la composition.

Polymères

La composition peut contenir en outre un ou plusieurs polymères hydrophiles.

Les polymères hydrophiles c'est-à-dire solubles ou dispersibles dans l'eau peuvent être choisis par exemple parmi les polymères carboxyvinyliques modifiés ou non, tels que les produits commercialisés sous les dénominations Carbopol (nom INCI : carbomer) et Pemulen (nom INCI : Acrylates/C10-30 akyl acrylate crosspolymer) par la société Noveon ; les polyacrylamides ; les polymères et copolymères d'acide 2-acrylamido 2-méthylpropane sulfonique, éventuellement réticulés et/ou neutralisés, comme le poly(acide 2-acrylamido 2-méthylpropane sulfonique) commercialisé par la société Hoechst sous la dénomination « Hostacerin AMPS » (nom INCI : ammonium polyacryldimethyltauramide) ; les copolymères anioniques réticulés d'acrylamide et d'AMPS, se présentant sous la forme d'une émulsion E/H, tels ceux commercialisés sous le nom de SEPIGEL 305 (nom C.T.F.A. : Polyacrylamide / C13-14 Isoparaffin / Laureth-7) et sous le nom de SIMULGEL 600 (nom C.T.F.A. : Acrylamide / Sodium acryloyldimethyltaurate copolymer / Isohexadecane / Polysorbate 80) par la société SEPPIC ; les biopolymères polysaccharidiques comme la gomme de guar et dérivés tels que la gomme de guar hydroxypropylée, les alginates, les celluloses modifiées ou non comme la méthylhydroxyéthylcellulose, l'hydroxypropylméthylcellulose ou encore l'hydroxyéthylcellulose ; et leurs mélanges. Ces polymères peuvent être présents en une quantité allant par exemple de 0,001 à 2 % en poids, de préférence de 0,05 à 1,5 % en poids par rapport au poids total de la composition.

Selon un mode préféré de réalisation de l'invention, les polymères sont choisis parmi le Carbopol 980 (BF Goodrich), Carbopol ETD 2001 (BF Goodrich), Synthalen K (3V Sigma), Carbopol 981 (BF Goodrich), Synthalen L (3V Sigma), Carbopol 1382 (BF Goodrich), Pemulen TR1 (BF Goodrich), Pemulen TR2 (BF Goodrich), Hostacerin AMPS (Clariant), plus spécialement le Carbopol 980 et l'Hostacerin AMPS.

Adjuvants

La composition selon l'invention peut contenir tout adjuvant habituellement utilisé dans les compositions cosmétiques ou dermatologiques.

Parmi les adjuvants susceptibles d'être contenus dans la phase aqueuse et/ou dans la phase huileuse des émulsions conformes à l'invention, on peut citer notamment les antioxydants, les émollients, les actifs cosmétiques ou dermatologiques, les parfums, les conservateurs, les charges, les séquestrants, les pigments, les colorants, ou tout autre ingrédient habituellement utilisé dans les domaines considérés.

Bien entendu, l'homme de l'art veillera à choisir le ou les éventuels composés à ajouter à la composition selon l'invention et leurs quantités de manière telle que les propriétés avantageuses attachées intrinsèquement à la composition conforme à l'invention ne soient pas, ou substantiellement pas, altérées par l'addition envisagée.

Comme actifs, on peut citer par exemple :

- les agents hydratants tels que par exemple le lactate de sodium ; les polyols, et en particulier la glycérine, le sorbitol, les polyéthylène glycols ; le mannitol ; les acides aminés ; l'acide hyaluronique ; la lanoline ; l'urée et les mélanges contenant de l'urée, tels que le NMF (« Natural Moisturising Factor ») ; la vaseline ; l'acide N-lauroyl pyrrolidone carboxylique et ses sels ; les acides gras essentiels ; les huiles essentielles ; et leurs mélanges.

- les actifs anti-vieillissement et les agents kératolytiques tels que les $\alpha$-hydroxy-acides et notamment les acides dérivés de fruits, comme les acides glycolique, lactique, malique, citrique, tartrique, mandélique, leurs dérivés et leurs mélanges ; les $\beta$-hydroxy-acides comme l'acide salicylique et ses dérivés tels que l'acide n-octanoyl-5-salicylique ou l'acide n-dodécanoyl-5-salicylique ; les $\alpha$-céto-acides comme l'acide ascorbique ou vitamine C et ses dérivés tels que ses sels comme l'ascorbate de sodium, l'ascorbylphosphate de magnésium ou de sodium ; ses esters comme l'acétate d'ascorbyle, le palmitate d'ascorbyle et le propionate d'ascorbyle, ou ses sucres comme l'acide ascorbique glycosylé, et leurs mélanges ; les $\beta$-céto-acides ; les rétinoïdes comme le rétinol (vitamine A) et ses esters, le rétinal, l'acide rétinoïque et ses dérivés, ainsi que les rétinoïdes décrits dans les documents FR-A-2,570,377, EP-A-199636, EP-A-325540, EP-A-402072 ; l'adapalène ; les caroténoïdes ; et leurs mélanges.

- les vitamines, comme les vitamines A et C indiquées ci-dessus, et aussi comme la vitamine E (tocophérol) et ses dérivés ; la vitamine B3 (ou vitamine PP ou niacinamide) et ses dérivés ; la vitamine B5 (ou panthénol sous ses différentes formes : D-panthénol, DL-panthénol), et ses dérivés et analogues, tels que le panthoténate de calcium, la panthétine, la pantothéine, l'éther de éthyl panthényl, l'acide pangamique, la pyridoxine, le pantoyl lactose, et les composés naturels en contenant tels que la gelée royale ; la vitamine D et ses analogues tels que ceux décrits dans le document WO-A-00/26167 ; la vitamine F ou ses analogues tels que les mélanges d'acides insaturés possédant au moins une double liaison et notamment les mélanges d'acide linoléique, d'acide linolénique et d'acide arachidonique, ou les composés en contenant ;

- les antibactériens et anti-séborrhéiques tels que l'acide salicylique, le 2,4,4'-trichloro-2'-hydroxy diphényl éther (ou triclosan), le 3,4,4'-trichlorobanilide (ou triclocarban), l'acide azélaïque, le peroxyde de benzoyle et les sels de zinc comme le lactate, le gluconate, le pidolate, le carboxylate, le salicylate et/ou le cystéate de zinc.

Les compositions de l'invention peuvent être utilisées sur toutes matières kératiniques telles que la peau, le cuir chevelu, les cheveux, les cils, les sourcils, les ongles ou les muqueuses. Elles peuvent être utilisées comme produits de soin de la peau, par exemple comme crèmes de protection, de traitement ou de soin pour le visage, pour les mains ou pour le corps, comme laits corporels de protection ou de soin de la peau, du cuir chevelu ou des muqueuses, ou comme produits d'hygiène, par exemple comme produits de nettoyage de la peau ou des muqueuses, ou encore comme produits capillaires ou comme produits solaires.

Les compositions peuvent constituer aussi des produits pour le maquillage de la peau et/ou des cheveux, par exemple en incorporant des pigments dans la composition pour constituer notamment des fonds de teint.

L'invention a encore pour objet l'utilisation cosmétique de la composition telle que définie ci-dessus comme produit de soin de la peau, comme produit d'hygiène, comme produit capillaire, comme produit solaire et comme produit de maquillage.

Un autre objet de l'invention est un procédé de traitement cosmétique d'une matière kératinique, telle que la peau, le cuir chevelu, les cheveux, les cils, les sourcils, les ongles ou les muqueuses, caractérisé par le fait qu'on applique sur la matière kératinique, une composition telle que définie ci-dessus.

Par ailleurs, quand les compositions selon l'invention ont la fluidité appropriée, elles peuvent servir aussi pour l'imprégnation de substrats insolubles dans l'eau pour constituer des articles (tels que lingettes) destinés au soin, au nettoyage et/ou au démaquillage de la peau, des cils et/ou des lèvres. Le substrat insoluble dans l'eau peut comprendre une ou plusieurs couches et il peut être choisi dans le groupe comprenant des matériaux tissés, des matériaux non-tissés, des mousses, des éponges, des ouates, en feuilles, boules ou films. Il peut s'agir notamment d'un substrat non tissé à base de fibres d'origine naturelle (lin, laine, coton, soie) ou d'origine synthétique (dérivés de cellulose, viscose, dérivés polyvinyliques, polyesters comme téréphtalate de polyéthylène, polyoléfines comme polyéthylène ou polypropylène, polyamides comme le Nylon, dérivés acryliques). Les non tissés sont décrits de façon générale dans RIEDEL « Nonwoven Bonding Methods & Materials », Nonwoven World (1987). Ces substrats sont obtenus selon les procédés usuels de la technique de préparation des non-tissés.

Quand le substrat est un non-tissé, on utilise de préférence un non-tissé épais, qui ne se met pas en boule et qui est assez solide pour ne pas se déliter et ne pas pelucher à l'application sur la peau. Il doit être absorbant, doux au moins sur une face pour le démaquillage des yeux en particulier. Comme non-tissés appropriés, on peut citer par exemple ceux commercialisés sous les dénominations Ultraloft 15285-01, Ultraloft 182-008, Ultraloft 182-010, Ultraloft 182-016 par la société BBA, Vilmed M1519 Blau, Vilmed M 1550 N et 112-132-3 par la société Freudenberg, celui commercialisé sous la dénomination Norafin 11601-010B par la société Jacob Holm Industries, les non tissés flockés commercialisés sous les dénominations Univel 109 et Univel 119 par la société Uni Flockage.

Par ailleurs, ce substrat peut comporter une ou plusieurs couches ayant des propriétés identiques ou différentes et avoir des propriétés d'élasticité, de douceur et autres appropriées à l'usage recherché. Les substrats peuvent comporter par exemple deux parties ayant des propriétés d'élasticité différentes comme décrit dans le document WO-A-99/13861 ou comporter une seule couche avec des densités différentes comme décrit dans le document WO-A-99/25318 ou comporter deux couches de textures différentes comme décrit dans le document WO-A-98/18441.

Les compositions imprégnées sur le substrat peuvent contenir tout composé approprié pour le but recherché, par exemple des tensioactifs moussants pour l'obtention de lingettes nettoyantes, ou des actifs de soin pour obtenir des lingettes de soin de la peau. On peut aussi les utiliser pour le maquillage de la peau, par exemple en imprégnant la lingette avec une composition contenant des pigments et pouvant constituer un fond de teint.

L'invention a donc aussi pour objet un article obtenu par imprégnation d'un substrat insoluble dans l'eau, avec une composition telle que définie ci-dessus.

L'invention a aussi pour objet l'utilisation de la composition telle que définie ci-dessus pour la préparation d'un article destiné au soin, au démaquillage, au nettoyage ou au maquillage de la peau, des lèvres et/ou des cils.

La composition selon l'invention peut être obtenue par tout procédé approprié. Selon un mode préféré de réalisation de l'invention, elle est de préférence préparée par le procédé consistant à mélanger à température ambiante (20 à 25°C) les composés de la phase aqueuse sauf l'alcool monohydrique, à préparer à température ambiante la phase huileuse en présence de l'alcool monohydrique, et à verser la phase huileuse dans la phase aqueuse sous agitation lente. On obtient une émulsion huile dans eau très fine caractérisée par une taille des globules huileux inférieure à 1000 nm et de préférence inférieure à 300 nm, par exemple de 100 à 300 nm, et mieux de 130 à 250 nm. et par un aspect de liquide opalescent à blanc.

L'invention a ainsi encore pour objet un procédé de préparation de la composition telle que définie ci-dessus, consistant à :

- mélanger à température ambiante l'eau, le ou les co-tensioactif(s) non ionique(s), le ou les co-tensioactif(s) anionique (s), et les composés hydrophiles sauf l'alcool monohydrique,
- mélanger, à température ambiante, les huiles, les actifs lipophiles, le ou les tensioactif(s) siliconé(s), et l'alcool monohydrique,
- verser la phase huileuse dans la phase aqueuse sous agitation lente.

Les exemples suivants illustrent l'invention sans présenter un caractère limitatif. Les quantités y sont des pourcentages en poids. Les composés sont indiqués en nom INCI ou en noms chimiques selon les cas.

Mode opératoire :

On a opéré dans ces exemples en mettant en oeuvre le mode opératoire suivant:

Phase A : on a solubilisé dans l'eau, les composés de la phase A sous agitation Rayneri équipé d'une turbine de type défloculeuse à la température ambiante.

Phase B : on a solubilisé les composés de la phase B dans l'alcool à la température ambiante.

On a ensuite versé la phase B dans la phase A sous agitation lente.

Exemple 1

| Phase A | |
|---|---|
| Eau | 81.55 % |
| Phenonip (mélange de conservateurs) | 0.5 % |
| Glycérine | 5 % |
| Dipropylène Glycol | 3 % |
| Sodium Lauroyl Glutamate | 0.03 % |
| Disodium stearoyl glutamate | 0.02% |
| Poloxamer 184 | 0.1 % |

Phase B
Ethanol                                                                                         8 %
Bis PEG/PPG-16/16 PEG/PPG-16/16 Dimethicone (et) Caprylic/Capric Triglyceride (Abil Care 85)   0.4 %
Pentaerythrityl Tetraethylhexanoate                                                              1.6 %

On a obtenu une émulsion huile dans eau très fine caractérisée par une taille des globules huileux de 220 nm, ayant l'aspect d'un liquide opalescent blanc.

Exemple 2 selon l'invention et exemples comparatifs 1 à 4

| Composition | Exemple 2 | Exemple comparatif 1 | Exemple comparatif 2 |
|---|---|---|---|
| Phase A | | | |
| Eau | 81,85 | 81,85 | 81,85 |
| Dipropylene Glycol | 3 | 3 | 3 |
| Glycérine | 3 | 3 | 3 |
| Stearoyl Glutamate disodique | 0,05 | 0,05 | 0,05 |
| Poloxamer 184 | 0,1 | 0,1 | 0,1 |
| Phenonip (conservateur) | 1 | 1 | 0,5 |
| Phase B | | | |
| Ethyl-2-hexyl palmitate | 1 | 1 | |
| Triglycerides d'acide Caprylique-Caprique | | | 1 |
| Bis-PEG/PPG-16/16 dimethicone / Caprylic/capric triglyceride (Abil care 85) | 0,5 | | |
| PEG-20 glyceryl triisostearate | | 0,5 | |
| Sucrose myristate | | | 0,5 |
| Ethanol | 10 | 10 | 10 |
| Résultat de la centrifugation (à 3000 tours/minute) | Emulsion fluide opalescente stable | Relargage d'huile 4 mm | Relargage d'huile 1 mm |

| | Exemple comparatif 3 | Exemple comparatif 4 |
|---|---|---|
| Phase A | | |
| Eau | 81,85 | 81,85 |
| Dipropylene Glycol | 3 | 3 |
| Glycérine | 3 | 3 |
| Stearoyl Glutamate disodique | 0,05 | 0,05 |
| poloxamer 184 | 0,1 | 0,1 |
| phenonip | 0,5 | 0,5 |

(suite)

| Phase B | | |
|---|---|---|
| Triglycerides d'acide Caprylique-Caprique | 1 | 1 |
| Polyglyceryl-10 isostearate | 0,5 | - |
| Lauroyl glycol hydroxypropyl ether | - | 0,5 |
| Ethanol | 10 | 10 |
| Résultat de la centrifugation (à 3000 tours/minute) | Relargage d'huile | Relargage d'huile et Déphasagge |

Les exemples comparatifs 1 à 4 montrent que l'utilisation de tensioactifs autres que les tensioactifs siliconés revendiqués dans la présente demande ne permet pas d'atteindre le but de l'invention et d'obtenir une émulsion fine stable.

Exemples 3 à 5 selon l'invention

| Composition | Exemple 3 selon l »invention | Exemple 4 selon l'invention | Exemple 5 selon l'invention |
|---|---|---|---|
| Phase A | | | |
| Eau | 81,85 | 81,85 | 81,85 |
| Dipropylene Glycol | 3 | 3 | 3 |
| Glycérine | 3 | 3 | 3 |
| Stearoyl Glutamate disodique | 0,05 | 0,05 | 0,05 |
| Poloxamer 184 | 0,1 | 0,1 | 0,1 |
| Phenonip (conservateur) | 0,5 | 1 | 0,5 |
| Phase B | | | |
| Ethyl2-hexyl palmitate | | 1 | |
| Triglycerides d'acide Caprylique-Caprique | 1 | | 1 |
| PEG/PPG-20/6 Dimethicone (Abil B88184) (P.M. 13000) | 0,5 | | |
| Bis-PEG/PPG-16/16 dimethicone / Caprylic/ capric triglyceride (Abil care 85) (P.M. 11000) | | 0,5 | |
| Bis-PEG/PPG-20/20 Dimethicone (Abil B8832) (P.M. 10000) | | | 0,5 |
| Ethanol | 10 | 10 | 10 |
| Résultat de la centrifugation (à 3000 tours/minute) | Emulsion fluide opalescente stable | Emulsion fluide opalescente stable | Emulsion fluide opalescente stable |

Exemples comparatifs 5 à 7

| Composition | Exemple comparatif 5 | Exemple comparatif 6 | Exemple comparatif 7 |
|---|---|---|---|
| Phase A | | | |
| Eau | 81,85 | 81,85 | 81,85 |
| Dipropylene Glycol | 3 | 3 | 3 |
| Glycérine | 3 | 3 | 3 |
| Stearoyl Glutamate disodique | 0,05 | 0,05 | 0,05 |
| Poloxamer 184 | 0,1 | 0,1 | 0,1 |
| Phenonip (conservateur) | 1 | 0,5 | 0,5 |
| Phase B | | | |
| Triglycerides d'acide Caprylique-Caprique | 1 | 1 | 1 |
| PEG-14 dimethicone (Abil B8843) (P.M. 5000) | 0,5 | | |
| PEG/PPG-14/4 Dimethicone (Abil B8851) (P.M. 5000) | | 0,5 | |
| PEG-12 Dimethicone (P.M. 3100) (Dow Corning 193 Fluid de Dow Corning) | | | 0,5 |
| Ethanol | 10 | 10 | 10 |
| Résultat de la centrifugation (à 3000 tours/minute) | Relargage d'huile (instabilité) | Relargage d'huile (instabilité) | Relargage d'huile (instabilité) |

Les exemples comparatifs 5 à 7 montrent que les tensioactifs siliconés de poids moléculaire inférieur à 10000 ne permettent pas d'atteindre le but de l'invention, c'est-à-dire d'obtenir une émulsion fine qui soit stable en centrifugation.

Exemples 6 et 7 selon l'invention et Exemples comparatifs 8 à 11

| Composition | Exemple 6 selon l'invention | Exemple 7 selon l'invention | Exemple comparatif 8 |
|---|---|---|---|
| Phase A | | | |
| Eau | 80,85 | 80,85 | 80,85 |
| Dipropylène Glycol | 3 | 3 | 3 |
| Glycérine | 3 | 3 | 3 |
| Stéaroyl Glutamate disodique | 0,05 | 0,05 | 0,05 |
| Poloxamer 184 | 0,1 | 0,1 | 0,1 |
| Phenonip (conservateur) | 0,5 | 1 | 0,5 |

(suite)

| Phase B | | | |
|---|---|---|---|
| Pentaerythrityl Tetraethylhexanoate | 2 | | |
| Phenyl trimethicone | | 2 | |
| Isoparaffine hydrogénée (P.M. = 350, IOB=0) (insoluble dans l'éthanol) | | | 2 |
| Bis-PEG/PPG-16/16 dimethicone / Caprylic/ capric triglyceride (Abil care 85) (P.M. 11000) | 0,5 | 0,5 | 0,5 |
| Ethanol | 10 | 10 | 10 |
| Résultat | Emulsion fluide opalescente stable | Emulsion fluide opalescente stable | Impossible de faire l'émulsion |

Les exemples comparatifs 9 à 11 sont analogues à l'exemple comparatif 8, mis à part que l'isoparaffine hydrogénée y est remplacée par d'autres huiles insolubles dans l'éthanol, respectivement par l'huile de macadamia (P.M. supérieur à 500, IOB < 0,1), l'isostéarate d'isostéaryle (P.M. = 350 et IOB = 0,086), le tétraisostéarate de pentaérythrityle (P.M. 640, IOB < 0,1). Dans tous les cas, il a été impossible de faire l'émulsion.

Exemples 8 à 10 selon l'invention

| Composition | Exemple 8 selon l'invention | Exemple 9 selon l'invention | Exemple 10 selon l'invention |
|---|---|---|---|
| Pentaerythrityl Tetraethylhexanoate | 1 | 1 | 1 |
| Phenyl trimethicone | 1 | 1 | 1 |
| Bis-PEG/PPG-16/16 dimethicone / Caprylic/ capric triglyceride (Abil care 85) (P.M. 11000) | 0,5 | 0,5 | 0,5 |
| Ethanol | 10 | 10 | 10 |
| Eau | 80,9 | 80,9 | 80,9 |
| Dipropylène Glycol | 3 | 3 | 3 |
| Glycérine | 3 | 3 | 3 |
| Stéaroyl Glutamate disodique | 0,02 | 0,02 | 0,02 |
| N-lauryl L-glutamate de sodium | 0,03 | 0,03 | 0,03 |
| Poloxamer 401 | 0,2 | | |
| Poloxamer 182 | | 0,2 | |
| Poloxamer 184 | | | 0,2 |
| Phenonip (conservateur) | 0,5 | 0,5 | 0,5 |
| Stabilité à 6 mois | Bonne | Bonne | bonne |

Exemple 11 selon l'invention et exemple comparatif 12

| Composition | Exemple 11 selon l'invention | Exemple comparatif 12 |
|---|---|---|
| Pentaerythrityl Tetraethylhexanoate | 1,6 | 1,6 |
| Bis-PEG/PPG-16/16 dimethicone / Caprylic/capric triglyceride (Abil care 85) (P.M. 11000) | 0,34 | 0,34 |
| Ethanol | 8 | 8 |
| Eau | 81,35 | 81,35 |
| Dipropylène Glycol | 3 | 3 |
| Glycérine | 5 | 5 |
| Stéaroyl Glutamate disodique | 0,02 | 0 |
| N-lauryl L-glutamate de sodium | 0,03 | 0 |
| Poloxamer 184 | 0,1 | 0,1 |
| Phenonip (conservateur) | 0,5 | 0,5 |
| Aspect au temps zéro | Emulsion fine et stable | Séparation de phases |

L'exemple comparatif 12 montre que la présence du co-tensioactif anionique est indispensable pour obtenir une émulsion stable.

**Revendications**

1. Composition sous forme d'émulsion huile-dans-eau, comprenant une phase huileuse dispersée dans une phase aqueuse, **caractérisée en ce qu'**elle contient (1) un système émulsionnant contenant au moins un tensioactif siliconé de poids moléculaire égal ou supérieur à 10000, au moins un co-tensioactif non ionique et au moins un co-tensioactif anionique, et (2) au moins un alcool monohydrique comportant de 2 à 6 atomes de carbone, et **en ce que** la ou les huiles de la phase huileuse sont solubles dans l'alcool monohydrique.

2. Composition selon la revendication 1, **caractérisée en ce que** le tensioactif siliconé est choisi parmi les polydiméthylsiloxanes comportant des groupements oxyéthylénés et oxypropylénés.

3. Composition selon l'une quelconque des revendications précédentes, **caractérisée en ce que** la quantité de tensioactif(s) siliconé(s) va de 0,05 à 4 % en poids par rapport au poids total de la composition.

4. Composition selon l'une quelconque des revendications précédentes, **caractérisée en ce que** le co-tensioactif non ionique est choisi parmi les polycondensats d'oxyde d'éthylène et d'oxyde de propylène.

5. Composition selon l'une quelconque des revendications précédentes, **caractérisée en ce que** le co-tensioactif non ionique est choisi parmi les polycondensats tribloc polyéthylène glycol / polypropylène glycol / polyéthylène glycol.

6. Composition selon l'une quelconque des revendications précédentes, **caractérisée en ce que** la quantité de co-tensioactif(s) non ionique(s) va de 0,01 à 0,5 % en poids par rapport au poids total de la composition.

7. Composition selon l'une quelconque des revendications précédentes, **caractérisée en ce que** le co-tensioactif anionique est choisi parmi les lipoaminoacides et leurs sels.

8. Composition selon l'une quelconque des revendications précédentes, **caractérisée en ce que** la quantité de tensioactif(s) anionique(s) va de 0,01 à 0,5 % en poids par rapport au poids total de la composition.

9. Composition selon l'une quelconque des revendications précédentes, **caractérisée en ce que** l'alcool monohydrique est l'éthanol.

10. Composition selon l'une quelconque des revendications précédentes, **caractérisée en ce que** la quantité d'alcool (s) monohydrique(s) va de 0.1 à 60 % en poids par rapport au poids total de la composition.

11. Composition selon l'une quelconque des revendications précédentes, **caractérisée en ce que** le rapport en poids de la quantité d'huile(s) sur la quantité de tensioactif(s) siliconé(s) va de 0,5 à 20.

12. Composition selon l'une quelconque des revendications précédentes, **caractérisée en ce que** le rapport en poids des quantités de tensioactif(s) siliconé(s) et d'huile(s) sur la quantité d'alcool(s) monohydrique(s) va de 0,01 à 1.

13. Composition selon l'une quelconque des revendications précédentes, **caractérisée en ce que** le rapport en poids de la quantité d'alcool(s) monohydrique(s) sur la quantité d'eau va de 0,01 à 0,2.

14. Composition selon l'une quelconque des revendications précédentes, **caractérisée en ce que** le rapport en poids de la quantité de co-tensioactif(s) anionique(s) sur la quantité de tensioactif(s) siliconé(s) va de 0,01 à 1.

15. Composition selon l'une quelconque des revendications précédentes, **caractérisée en ce qu'**elle contient un ou plusieurs polyols.

16. Composition selon l'une quelconque des revendications précédentes,, **caractérisée en ce que** les huiles sont choisies soit parmi les huiles ayant un poids moléculaire inférieur ou égal à 350, soit parmi les huiles ayant un poids moléculaire supérieur à 350 et une valeur d'IOB supérieure ou égale à 0,1.

17. Composition selon l'une quelconque des revendications précédentes,, **caractérisée en ce que** les huiles sont choisies parmi les phényltriméthicones, les esters du pentaérythritol, l'octyldodécanol, l'isohexadecane, l'isododecane, les cyclopolydiméthylsiloxanes, et leurs mélanges.

18. Composition selon l'une quelconque des revendications précédentes,, **caractérisée en ce que** la quantité de phase huileuse va de 0,5 à 10 % en poids par rapport au poids total de la composition.

19. Composition selon l'une quelconque des revendications précédentes,, **caractérisée en ce que** la taille moyenne des globules de phase huileuse va de 50 à 1000 nm.

20. Utilisation cosmétique de la composition selon l'une quelconque des revendications précédentes, comme produit de soin de la peau, comme produit d'hygiène, comme produit capillaire, comme produit solaire et comme produit de maquillage.

21. Procédé de traitement cosmétique d'une matière kératinique, **caractérisé en ce qu'**on applique sur la matière kératinique, une composition selon l'une quelconque des revendications 1 à 19.

22. Article obtenu par imprégnation d'un substrat insoluble dans l'eau, avec une composition selon l'une quelconque des revendications 1 à 19.

23. Procédé de préparation de la composition selon l'une quelconque des revendications 1 à 19, consistant à :

- mélanger à température ambiante l'eau, le ou les co-tensioactif(s) non ionique(s), le ou les co-tensioactif(s) anionique(s), et les composés hydrophiles sauf l'alcool monohydrique,
- mélanger, à température ambiante, les huiles, les actifs lipophiles, le ou les tensioactif(s) siliconé(s), et l'alcool monohydrique,
- verser la phase huileuse dans la phase aqueuse sous agitation lente.

**Office européen des brevets**

## RAPPORT DE RECHERCHE EUROPEENNE

Numéro de la demande

EP 07 10 6769

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| A | BERND SCHRÄDER ET AL: "O/W Emulgator, O/W Emulsion und deren Verwendung" RESEARCH DISCLOSURE, MASON PUBLICATIONS, HAMPSHIRE, GB, vol. 473, no. 2, septembre 2003 (2003-09), XP007132816 ISSN: 0374-4353 * le document en entier * ----- | 1-23 | INV. A61K8/06 A61K8/894 A61K8/90 A61K8/44 A61K8/34 A61Q1/00 A61Q5/02 A61Q19/10 A61Q19/00 |
| A | EP 0 516 547 A1 (OREAL [FR]) 2 décembre 1992 (1992-12-02) * le document en entier * ----- | 1-23 | |
| A | EP 0 076 146 A2 (PROCTER & GAMBLE [US]) 6 avril 1983 (1983-04-06) * exemples I-V * ----- | 1-23 | |

DOMAINES TECHNIQUES RECHERCHES (IPC)

A61K
A61Q

Le présent rapport a été établi pour toutes les revendications

| Lieu de la recherche | Date d'achèvement de la recherche | Examinateur |
|---|---|---|
| Munich | 8 octobre 2007 | NOPPER-JAUNKY, A |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 07 10 6769

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

08-10-2007

| Document brevet cité au rapport de recherche | | Date de publication | Membre(s) de la famille de brevet(s) | | Date de publication |
|---|---|---|---|---|---|
| EP 0516547 | A1 | 02-12-1992 | CA | 2069571 A1 | 28-11-1992 |
| | | | DE | 69202102 D1 | 24-05-1995 |
| | | | DE | 69202102 T2 | 26-10-1995 |
| | | | ES | 2072112 T3 | 01-07-1995 |
| | | | FR | 2676921 A1 | 04-12-1992 |
| | | | JP | 3537836 B2 | 14-06-2004 |
| | | | JP | 5262620 A | 12-10-1993 |
| EP 0076146 | A2 | 06-04-1983 | CA | 1191789 A1 | 13-08-1985 |
| | | | DE | 3278388 D1 | 01-06-1988 |
| | | | JP | 1737959 C | 26-02-1993 |
| | | | JP | 4020884 B | 07-04-1992 |
| | | | JP | 58131910 A | 06-08-1983 |
| | | | MX | 160901 A | 12-06-1990 |
| | | | US | 4421769 A | 20-12-1983 |

EPO FORM P0460

Pour tout renseignement concernant cette annexe : voir Journal Officiel de l'Office européen des brevets, No.12/82

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 728460 A
- EP 780114 A
- EP 1010413 A
- EP 1010414 A
- EP 1010415 A
- EP 1010416 A
- EP 1013338 A
- EP 1016453 A
- EP 1018363 A
- EP 1020219 A
- EP 1025898 A
- EP 1172077 A
- EP 1297824 A
- EP 0774482 A
- EP 985404 A
- FR 2570377 A
- EP 199636 A
- EP 325540 A
- EP 402072 A
- WO 0026167 A
- WO 9913861 A
- WO 9925318 A
- WO 9818441 A

**Littérature non-brevet citée dans la description**

- **A. FUJITA.** Prediction of organic compounds by a conceptionnal diagram. *Pharm. Bull,* 1954, vol. 2, 163-173
- **FUJITA.** Organic Analysis. Kaniya Shoten, 1930
- *Prediction of Organic Compounds and Organic Conceptional Diagram,* 1957, 719-725
- **FUJITA ; AKATSUKA.** Systematic Organic Qualitative Analysis (Book of Purified Substances. Kazama Shoten, 1970, 487
- **KODA.** Organic Conceptional Diagram, Its Fundamentals and applications. Sankyo Shuppan, 1984, 227
- **YAGUCHI.** Design of Emulsion Formulations by use of Organic Conceptional Diagram. Nippon Emulsion K.K, 1985, 98
- **R. H. EWELL ; J. M. HARRISON ; L. BERG.** *Ind. Eng. Chem.,* 1944, vol. 36, 871
- **RIEDEL.** Nonwoven Bonding Methods & Materials. Nonwoven World, 1987